# EUROPEAN PATENT SPECIFICATION

(11) **EP 2 012 763 B1**
(45) Date of publication and mention of the grant of the patent: **23.03.2011**
(21) Application number: 07724561.1
(22) Date of filing: 25.04.2007
(51) Int. Cl.: A61K 31/137, A61K 31/192, A61K 31/196, A61P 29/00

(54) **PHARMACEUTICAL COMBINATION COMPRISING 3- (3-DIMETHYLAMIN0-1-ETHYL-2-METHYL-PR0PYL) -PHENOL AND AN NSAID**
PHARMAZEUTISCHE KOMBINATION MIT 3- (3-DIMETHYLAMINO-1-ETHYL-2-METHYL-PROPYL) PHENOL UND EINEM NSAR
COMBINAISON PHARMACEUTIQUE COMPRENANT 3- (3-DIMÉTHYLAMINO-1-ÉTHYL-2-MÉTHYL-PROPYL) -PHÉNOL ET UN AINS

(30) Priority: 28.04.2006 EP 06008850
(43) Date of publication of application: 14.01.2009
(73) Proprietor: Grünenthal GmbH, 52078 Aachen (DE)
(72) Inventor: SCHIENE, Klaus, 41363 Jüchen (DE); BLOMS-FUNKE, Petra, 52146 Würselen (DE)
(74) Representative: Brosch, Oliver
(86) International application number: PCT/EP2007/003631
(87) International publication number: WO 2007/128412

(56) References cited:
- WO-A-2004/047823
- DE-A1- 4 426 245
- "THE MERCK MANUAL OF DIAGNOSIS AND THERAPY; 17TH Edition" 1999, MERCK RESEARCH LABORATORIES , NEW JERSEY, USA , XP002401271 pages1363-1365 Ch. 167 "PAIN"

## Description

The present invention relates to a combination comprising as components (a) the compound 3-(3-Dimethylamino-l-ethyl-2-methyl-propyl)-phenol, and (b) one or more non-steroidal anti-inflammatory drugs (NSAIDs); a pharmaceutical salt comprising said components; a compound derived from said components; a pharmaceutical formulation and a dosage form comprising said combination, salt, or compound; as well as a method of treating pain, e.g. chronic or acute pain, in a mammal characterized in that components (a) and (b) are administered simultaneously or sequentially to a mammal, wherein component (a) may be administered before or after component (b) and wherein components (a) or (b) are administered to the mammal either via the same or a different pathway of administration.

The treatment of chronic and acute pain conditions is extremely important in medicine. There is currently a worldwide demand for additional, not exclusively oploid-based, but highly effective pain treatment. The urgent need for action for patient-oriented and purposeful treatment of pain conditions, this being taken to mean the successful and satisfactory treatment of pain for the patient, is documented in the large number of scientific papers which have recently appeared in the field of applied analgesics and fundamental research work on nociception.

Even if the analgesics that are currently used for treating pain, for example opioids, NA- and 5HT-reuptake inhibitors, NSAIDS and COX inhibitors, are analgesically effective, side effects nevertheless sometimes occur. WO 2004/047823 describes substance combinations comprising certain analgesics including 1-phenyl-3-dimethylamino-propane compounds and COX-11 Inhibitors, which show super-additive effects upon administration. Due to the super-additive effect the overall dose and accordingly the risk of undesired side effects can be reduced.

DE 44 26 245 A1 discloses 1-Phenyl-3-dimethylamino-propane compounds having pharmacological activity.

"The Merck Manual of Diagnosis and Therapy"; 17th edition, 1999, Merck Research Laboratories, New Jersey, USA describes the use of NSAIDS for the treatment of pain.

Thus, it was an object of the present invention to find further combinations that are suitable for the treatment of pain and which preferably exhibit fewer undesired side effects compared to its individual components, if administered in effective doses.

It has been found that a pharmaceutical combination comprising (a) the compound 3-(3-Dimethylamino-1-ethyl-2-methyl-propyl)-phenol, and (b) at least one non-steroidal anti-inflammatory drug (NSAID) exhibits an analgesic effect. If these components are present in the composition in such a weight ratio that a synergistic effect is observed after administration to the patients, the overall administered dose may be lowered, so that fewer undesired side-effects will occur.

Accordingly, the present invention relates to a combination comprising as components
(a) 3-(3-Dimethylamino-1-ethyl-2-methyl-propyl)-phenol of formula (I) 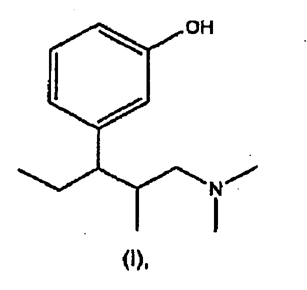 optionally in form of one of its pure stereoisomers, in particular an enantiomer or a diastereomer, a racemate or in form of a mixture of its stereoisomers, in particular enantiomers and/or diastereomers In any mixing ratio, or any corresponding acid addition salt thereof, or any solvate thereof, and
(b) one or more non-steroidal anti-inflammatory drugs (NSAIDs) selected from the group consisting of Diclofenac, Diclofenac-Sodium, Dipyrone (Metamizol), Metamizol-Sodium, Ibuprofen, Ketoprofen, (+)-Ibuprofen and (-)-Ibuprofen.

In an embodiment of the inventive combination component (a) is selected from
(1R,2R)-3-(3-Dimethylamino-1-ethyl-2-methyl-propyl)-phenol,
(1S,2S)-3-(3-Dimethylamino-1-ethyl-2-methyl-propyl)-phenol,
(1R,2S)-3-(3-Dimethylamino-1-ethyl-2-methyl-propyl)-phenol,
(1S,2R)-3-(3-Dimethylamino-1-ethyl-2-methyl-propyl)-phenol, and any mixture thereof.

In another embodiment of the inventive combination component (a) Is selected from
(1R,2R)-3-(3-Dimethylamino-1-ethyl-2-methyl-propyl-phenol, and
(1S,2S)-3-(3-Dimothylamlno-1-ethyl-2-methyl-propyl-phenol, and any mixture thereof.

In yet another embodiment the inventive combination comprises
(a) the compound (1R,2R)-3-(3-Dimethylamino-1-ethyl-2-methyl-p propyl)-phenol of formula (l'). 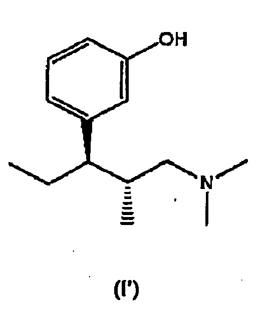 or an acid addition salt thereof, and
(b) one or more non-steroidal anti-inflammatory drugs (NSAIDs) selected from the group consisting of Diclofenac, Diclofenac-Sodium, Dipyrone (Metamizol), Metamizol-Sodium, Ibuprofen, Ketoprofen, (+)-Ibuprofen and (-)-Ibuprofen.

The compound 3-(3-Dimethylamino-1-ethyl-2-methyl-propyl)-phenol of formula (I), its stereoisomers and corresponding salts thereof as well as methods for their preparation are well known, for example, from US 6,248,737 B1. The respective parts of the description are hereby incorporated by reference and form part of the present disclosure.

The definition of component (a) as used herein includes the compound 3-(3-Dimethylamino-1-ethyl-2-methyl-propyl)-phenol and its stereoisomers in any possible form, thereby particularly including solvates, acid addition salts and corresponding solvates and polymorphs thereof.

If any of the components, particularly component (a), is present as mixture of enantiomers, such a mixture may contain the enantiomers in racemic or non-racemic form. A non-racemic form could, for example, contain the enantiomers in a ratio of 60:40, 70:30, 80:20 or 90:10.

The compound 3-(3-Dimethylamino-1-ethyl-2-methyl-propyl)-phenol and its stereoisomers according to component (a) may be present in the inventive pharmaceutical composition in form of an acid addition salt, whereby any suitable acid capable of forming such an addition salt may be used.
The conversion of the compound 3-(3-Dimethylamino-1-ethyl-2-methyl-propyl)-phenol into a corresponding addition salt via reaction with a suitable acid may be effected in a manner well known to those skilled in the art. Suitable acids include but are not limited to hydrochloric acid, hydrobromic acid, sulfuric acid, methanesulfonic acid, formic acid, acetic acid, oxalic acid, succinic acid, tartaric acid, mandelic acid, fumaric acid, lactic acid, citric acid, glutamic acid and/or aspartic acid. Salt formation is preferably effected in a solvent, for example diethyl ether, diisopropyl ether, alkyl acetates, acetone and/or 2-butanone. Moreover, trimethylchlorosilane in aqueous solution is also suitable for the preparation of hydrochlorides.

It is known to those skilled in the art that the analgesic action of NSAIDs is due to the inhibition of the enzymatic production of prostaglandins, wherein Cyclooxygenase (COX) is the key enzyme in the conversion of arachidonic acid derived from lipids of the cell membrane to prostaglandins and other eicosanoids. COX exists in two different isoforms characterized by different expression patterns. COX-I is constitutively expressed in many cells of the body and responsible mainly for the production of eicosanoids serving normal physiological functions. COX-II expression is induced during inflammation and also COX-II is expressed in the central nervous system.

The term non-steroidal anti-inflammatory drug as used herein designates compounds showing essentially COX-I specific inhibition selective COX-I or mixed COX-I/II inhibition, so that selective COX-II Inhibitors are not encompassed. The term non-steroidal anti-inflammatory drugs of component (b) as used herein includes any possible form of these NSAIDs, particularly including stereoisomers such as enantiomers, solvates, salts and corresponding solvates and polymorphs thereof. For example, the term Ibuprofen as used herein particularly Includes its racemic mixtures, its non-racemic mixtures, and its pure stereoisomer such as (S)-(+)-Ibuprofen and the term Diclofenac as used herein may particularly include its salt Diclofenac-sodium.

Non-steroidal and-inflammatory drugs as well as processes for their preparation are well known in the art, for example from E. Friderichs et al. "Analgesics and Antipyretics", Ullmann's Encyclopedia of Industrial Chemistry, Sixth Edition, Wiley-VCH Verlag GmbH, Germany 2000, pages 1-22 and H. Buschmann, T. Christoph, E. Friderichs, C, Maul, B. Sundermann, "Analgesics- From Chemistry and Pharmacology to Clinical Application", 2002, Part II, Wiley-VCH Verlag, Germany. The respective parts of said literature descriptions are incorporated by reference and form part of the present disclosure.

In another embodiment of the inventive combination component (b) is selected from the group consisting of Diclofenac, Diclofenac-Sodium, Dipyrone (Metamizol), Metamizol-Sodium, Ibuprofen and Ketoprofen

In yet another embodiment of the inventive combination component (b) is selected from the group consisting of Diclofenac and Ibuprofen.

In still another embodiment of the inventive combination component (b) is selected from the group consisting of Metamizol, Metamizol-Sodium and Ketoprofen

Other specific embodiments of the present invention are combinations comprising
(a) (1R,2R)-3-(3-Dimethylamino-1-ethyl-2-methyl-propyl)-phenol, or the hydrochloride addition salt thereof, and (b) Diclofenac and
(a) (1 R,2R)-3-(3-Dimethylamino-1-ethyl-2-methyl-propyl)-phenol, or the hydrochloride addition salt thereof, and (b) Ibuprofen.

Further specific embodiments of the present invention are combinations selected from the group consisting of
(a) (1R,2R)-3-(3-Dimethylamino-1-ethyl-2-methyl-propyl)-phenol, or the hydrochloride addition salt thereof, and (b) Diclofenac-Sodium,
(a) (1R,2R)-3-(3-Dimethylamino-1-ethyl-2-methyl-propyl)-phenol, or the hydrochloride addition salt thereof, and (b) Ketoprofen and
(a) (1R,2R)-3-(3-Dimethylamino-1-ethyl-2-methyl-propyl)-phenol, or the hydrochloride addition salt thereof, and (b) Metamizol-Sodium.

In case Diclofenac or Ibuprofen form part of the inventive combination, these substances may be administered in their usual daily dosage.
Preferably, the daily amount of Diclofenac administered to a patient is 25 to 300 mg, particularly preferably the amount is 35 to 200 mg, yet more preferably 50 to 150 mg.
Preferably the daily amount of Ibuprofen administered to a patient is 300 to 2400 mg, particularly preferably the amount is 350 to 1600 mg, yet more preferably 400 to 1200 mg.
In case Ketoprofen or Metamizol-Sodium form part of the inventive combination, these substances may be administered in their usual daily dosage.
Preferably, the daily amount of Ketoprofen administered to a patient is 1 to 250 mg, preferably 5 to 200 mg.
Preferably, the daily amount of Metamizol-Sodium administered to a patient is 1 to 4500 mg, preferably 5 to 4000 mg.
(1R,2R)-3-(3-Dimethylamino-1-ethyl-2-methyl-propyl)-phenol is preferably administered to a patient in a daily dosage of 25 to 1000 mg, particularly preferably in a dosage of 50 to 800 mg, more particularly preferably in a dosage of 100 to 600 mg.

In another embodiment of the present invention the inventive combination may contain components (a) and (b) essentially in an equieffective ratio.

In yet a further embodiment of the inventive combination components (a) and (b) are present in such a weight ratio that the resulting composition will exert a synergistic effect upon administration to a patient. Suitable weight ratios can be determined by methods well known to those skilled in the art, e.g. via the Randall-Selitto test described below.

Both components (a) and (b) may also be present in the inventive combination in ratios deviating from the equieffective ratio. For, example, each of the components could be present in a range from 1/5 of the equieffective amount to 5 times the equieffective amount, preferably 1/4 to 4, more preferably 1/3 to 3, yet more preferably 1/2 to 2 of the equieffective amount.

In another embodiment of the present invention the components (a) and (b) can be administered in a specific dosage regimen to treat pain, for example, chronic pain or acute pain. Components (a) and (b) may be administered simultaneously or sequentially to one another, in each case via the same or different administration pathways. Another aspect of the present invention is therefore a method of treating pain, e.g. chronic or acute pain, characterized in that components (a) and (b) are administered simultaneously or sequentially to a mammal, wherein component (a) may be administered before or after component (b) and wherein components (a) or (b) are administered to the mammal either via the same or a different pathway of administration. Suitable pathways of administrations include but are not limited to oral, intravenous, intraperitoneal, transdermal, intrathekal, intramuscular, intranasal, transmucosal, subcutaneous, or rectal administration.

Some non-steroidal anti-inflammatory drugs such as Diclofenac and Ibuprofen have acidic groups such as carboxy groups and may be used as such to form acid addition salts with the compound 3-(3-Dimethylamino-1-e1hyl-2-methyl-propyl)-phenol of formula (I), thereby incorporating both components (a) and (b) in one and the same salt.

Thus, in another embodiment of the present invention the inventive combination comprises components (a) and (b) in form of a salt formed from these two components. Such a salt formation may be partial, i.e. the inventive composition comprises one or both of these components also in their non-salt form, or the salt formation may essentially be complete.

Accordingly, in another aspect the present invention relates to a salt formed from
(a) at least one 3-(3-Dimethylamino-1-ethyl-2-methyl-propyl-phenol) of formula (I), 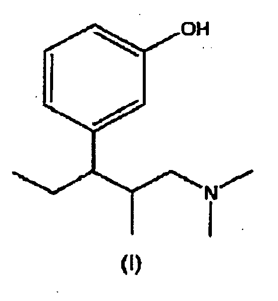 optionally in form of one of its pure stereoisomers, in particular an enantiomer for a diastereomer, a racemate or in form of a mixture of its stereoisomers, in particular enantiomers and/or diastereomers in any mixing ratio, or any solvate thereof, and
(b) one or more acidic non-steroidal anti-inflammatory drugs (NSAIDs) having a group that is capable of forming a salt with component (a) selected from the group consisting of Diclofenac, Dipyrone (Metamizol), Ibuprofen, Ketoprofen, (+)-Ibuprofen and (-)-Ibuprofen.

In the pharmaceutical salt, the cationic salt component is formed from (a) the compound 3-(3-Dimethylamino-1-ethyl-2-methyl-propyl)-phenol of formula (I) and the anionic salt component is formed from (b) an acidic non-steroidal anti-inflammatory drug selected from the group consisting of Diclofenac, Dipyrone (Metamizol), Ibuprofen, Ketoprofen, (+)-Ibuprofen and (-)-Ibuprofen.

In one embodiment of the inventive pharmaceutical salt component (a) is selected from
(1R,2R)-3-(3-Dimethylamlno-1-ethyl-2-methyl-propyl)-phenol,
(1S,2S)-3-(3-Dimethylamino-1-ethyl-2-methyl-propyl)-phenol,
(1R,2S)-3-(3-Dimethylamino-1-ethyl-2-methyl-propylrphenol,
(1S,2R)-3-(3-Dimethylamino-1-ethyl-2-methyl-propyl-phenol and any mixture thereof.

In yet another embodiment of the inventive pharmaceutical salt component (a) is selected from
(1R,2R)-3-(3-Dimethylamino-1-ethyl-2-methyl-propyl)-phenol,
(1S,2S)-3-(3-Dimethylamino-1-ethyl-2-methyl-propyl)-phenol, and any mixture thereof.

In a further embodiment of the inventive pharmaceutical salt component (a) is the compound (1R,2R)-3-(3-Dimethylamino-1-ethyl-2-methyl-propyl)-phenol of formula (I')

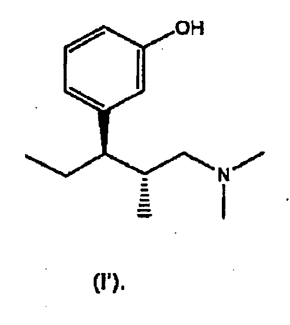

In another embodiment of the inventive pharmaceutical salt the acidic non-steroidal anti-inflammatory drug is selected from the group consisting of Diclofenac, Dipyrone (Metamizol), Ibuprofen and Ketoprofen .

Particular embodiments of the inventive pharmaceutical salts are the addition salts formed from
(1R,2R)-3-(3-Dimethylamino-1-ethyl-2-methyl-propyl)-phenol and Diclofenac, and
(1R,2R)-3-(3-Dimethylamino-1-ethyl-2-methyl-propyl)-phenol and Ibuprofen.

Further particular embodiments of the inventive pharmaceutical salts are the addition salts formed from
(1R,2R)-3-(3-Dimethylamino-1-ethyl-2-methyl-propyl)-phenol and Ketoprofen, and
(1R,2R)-3-(3-Dimethylamino-1-ethyl-2-methyl-propyl)-phenol and Metamizol.

The 3-(3-Dimethylamino-1-ethyl-2-methyl-propyl)-phenol compound of component (a) and the NSAID component (b) may also be linked to one another, for example, via a covalent linkage. Such a covalent linkage may, for example, be obtained from the phenolic hydroxy group of component (a) and a carboxy group of an NSAID according to component (b), whereby an ester linkage is obtained.

Accordingly, in yet another aspect the present invention relates to a compound of general formula (I") 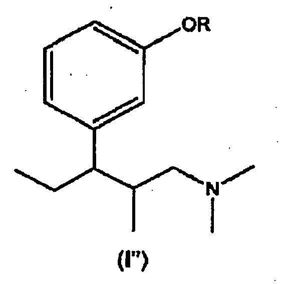 wherein R is a fragment of a non-steroidal anti-inflammatory drug (NSAID) that is attached to the oxygen atom via a covalent bond,
optionally in form of one of its pure stereoisomers, in particular an enantiomer or a diastereomer, a racemate or in form of a mixture of its stereoisomers, in particular enantiomers and/or diastereomers in any mixing ratio, or any corresponding salt thereof, or any solvate thereof selected from the group consisting of Diclofenac, Dipyrone (Metamizol), Ibuprofen, Ketoprofen, (+)-Ibuprofen and (-)-Ibuprofen.

In one embodiment the compound of formula I" is derived from
(1R,2R)-3-(3-Dimethylamino-1-ethyl-2-methyl-propyl)-phenol,
(1S,2S)-3-(3-Dimethylamino-1-ethyl-2-methyl-propyl)-phenol,
(1R,2S)-3-(3-Dimethylamino-1-ethyl-2-methyl-propyl)-phenol,
(1S,2R)-3-(3-Dimethylamino-1-ethyl-2-methyl-propyl)-phenol and any mixture thereof.

In another embodiments the compound of formula I" is derived from
(1R,2R)-3-(3-Dimethylamino-1-ethyl-2-methyl-propyl)-phenol,
(1S,2S)-3-(3-Dimethylamino-1-ethyl-2-methyl-propyl)-phenol, and any mixture thereof.

In yet another embodiment the compound of formula I" is derived from the compound (1R,2R)-3-(3-Dimethylamino-1-ethyl-2-methyl-propyl)-phenol of formula (I')

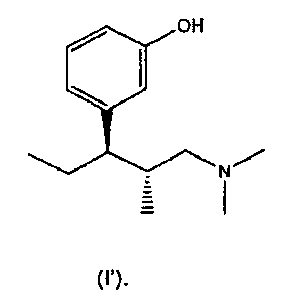

In another embodiment the compound of formula I" is derived from an acidic non-steroidal anti-inflammatory drug selected from the group consisting of Diclofenac, Dipyrone (Metamizol), Ibuprofen, and Ketoprofen .

The inventive combinations, the inventive pharmaceutical salts as well as the inventive compounds of formula (I") are toxicologically safe and are therefore suitable for the treatment of mammals, particularly humans including infants, children and grown-ups.

Thus, in a further aspect the present invention relates to a pharmaceutical composition comprising an inventive combination as described herein and/or a pharmaceutical salt as described herein and/or a compound of formula (I") as described herein and one or more auxiliary agents.

In a further aspect the present invention relates to a pharmaceutical dosage form comprising an inventive combination as described herein and/or a pharmaceutical salt as described herein and/or a compound of formula (I") as described herein and one or more auxiliary agents.

In one embodiment the inventive pharmaceutical dosage form comprises additionally caffeine.

In one embodiment, the inventive pharmaceutical dosage form is suitable for being administered orally, intravenously, intraperitoneally, transdermally, intrathekally, intramuscularly, intranasally, transmucosally, subcutaneously, or rectally.

The inventive formulations and dosage forms may contain auxiliary agents, for example, carriers, fillers, solvents, diluents, colorants and/or binders. The selection of auxiliary agents and of the amounts of the same to be used depends, for example, on how the drug is to be administered, e.g. orally, intravenously, intraperitoneally, intradermally, intramuscularly, intranasally or locally, for example for infections of the skin, of the mucous membranes or of the eye.

Suitable auxiliary agents in the context of this invention are any substances known to a person skilled in the art useful for the preparation of galenical formulations. Examples of suitable auxiliary agents include but are not limited to: water, ethanol, 2-propanol, glycerol, ethylene glycol, propylene glycol, polyethylene glycol, polypropylene glycol, glucose, fructose, lactose, saccharose, dextrose, molasses, starch, modified starch, gelatine, sorbitol, inositol, mannitol, microcrystalline cellulose, methyl cellulose, carboxymethyl cellulose, cellulose acetate, shellac, cetyl alcohol, polyvinyl pyrrolidone, paraffins, waxes, natural and synthetic gums, acacia gum, alginates, dextran, saturated and unsaturated fatty acids, stearic acid, magnesium stearate, zinc stearate, glycerol stearate, sodium lauryl sulphate, edible oils, sesame oil, coconut oil, peanut oil, soybean oil, lecithin, sodium lactate, polyoxyethylene and polypropylene fatty acid ester, sorbitan fatty acid ester, sorbic acid, benzoic acid, citric acid, ascorbic acid, tannic acid, sodium chloride, potassium chloride, magnesium chloride, calcium chloride, magnesium oxide, zinc oxide, silicon dioxide, titanium oxide, titanium dioxide, magnesium sulphate, zinc sulphate, calcium sulphate, potash, calcium phosphate, dicalcium phosphate, potassium bromide, potassium iodide, talcum, kaolin, pectin, crosspovidone, agar and bentonite.

Pharmaceutical formulations (dosage forms) in the form of tablets, effervescent tablets, chewing tablets, dragees, capsules, drops, juices or syrups are, for example, suitable for oral administration. Oral pharmaceutical formulations may also be in the form of multiparticulates such as granules, pellets, spheres, crystals and the like, optionally compressed into a tablet, filled into a capsule, filled into a sachet or suspended in a suitable liquid medium. Oral pharmaceutical formulations may also be equipped with an enteric coating.

Pharmaceutical formulations that are suitable for parenteral, topical and inhalative administration include but are not limited to solutions, suspensions, easily reconstitutable dry preparations and sprays.

Suppositories are a suitable pharmaceutical formulation for rectal administration. Formulations in a deposit, in dissolved form, for example, in a patch optionally with the addition of agents to promote skin penetration, are examples of suitable formulations for percutaneous administration.

One or both of the components (a) and (b) and/or the inventive pharmaceutical salt and/or the inventive compound of formula (I") may be present in the inventive pharmaceutical formulation at least partially in controlled-release form. Moreover, any controlled release/immediate release combination of said components may also be present in the inventive pharmaceutical formulation. For example, one or both of the components may be released from the inventive formulations with a certain delay, e.g. if administered orally, rectally or percutaneously. Such formulations are particularly useful for "once-daily" or "twice-daily" preparations, which only have to be taken once a day, respectively, twice a day. Suitable controlled-release materials are well known to those skilled in the art.

The inventive pharmaceutical formulations may be produced using materials, means, devices and processes that are well known in the prior art of pharmaceutical formulations, as described for example in "Remington's Pharmaceutical Sciences", A.R. Gennaro (ed.), 17th edition, Mack Publishing Company, Easton, Pa. (1985), in particular in part 8, chapters 76 to 93.

In order to obtain a solid pharmaceutical formulation such as a tablet, for example, the components of the pharmaceutical composition may be granulated with a pharmaceutical carrier, for example conventional tablet ingredients such as corn starch, lactose, saccharose, sorbitol, talcum, magnesium stearate, dicalcium phosphate or pharmaceutically acceptable gums, and pharmaceutical diluents, for example water, in order to form a solid composition that contains the components in homogeneous distribution. The term "homogeneous distribution" is taken to mean that the components are distributed uniformly over the entire composition, so that said composition may easily be divided into equally effective unit dose forms, such as tablets, pills or capsules. The solid composition is then divided into unit dose forms. The tablets or pills of the pharmaceutical composition according to the invention may also be coated or compounded in a different manner, in order to provide a dose form with a controlled release.

If one of the components, e.g. component (b), is to be released prior to the other component, for example at least 30 minutes or 1 hour beforehand, pharmaceutical formulations having a corresponding release profile may be prepared. An example of such a formulation is an osmotically driven release system for achieving a delayed release of component (a) via a coating that itself contains component (b) which is accordingly released earlier. In a release system of this kind, which is particularly suitable for oral administration, at least part, and preferably all, of the surface of the release system, preferably those parts that will come into contact with the release medium, is/are semipermeable, preferably equipped with a semipermeable coating, so the surface(s) is/are permeable to the release medium, but substantially, preferably entirely, impermeable to the active ingredient, component (a), the surface(s) and/or optionally the coating comprising at least one opening for releasing the active ingredient, component (a). Moreover, precisely that/those surface(s) that is/are in contact with the release medium is/are provided with a coating containing and releasing the other component, component (b). This is preferably taken to mean a system in tablet form comprising a release opening, an osmotic pharmaceutical composition core, a semipermeable membrane and a polymer portion that exerts pressure upon swelling. A suitable example of this kind of system is the system distributed by ALZA Corporation, USA under the tradenames OROS®, in particular, the OROS® Push-Pull™ System, the OROS® Delayed Push-Pull™ System, the OROS® Multi-Layer Push-Pull™ system, the OROS® Push-Stick System and also, in specific cases, the L-OROS™.

Embodiments and examples of osmotically driven release systems are, for example, disclosed in US patents 4,765,989, 4,783,337 and 4,612,008, all of the respective contents of which are hereby incorporated by reference and form part of the disclosure of the present invention.

A further example of a suitable pharmaceutical formulation is a gel-matrix tablet, such as the products developed by Penwest Pharmaceuticals (for example, under TimeRX). Suitable examples are provided in US patents 5,330,761, 5,399,362, 5,472,711 and 5,455,046, all of the respective contents of which are hereby incorporated by reference and form part of the disclosure of the present invention. Particularly suitable is a retarding matrix formulation, with an inhomogeneous distribution of the pharmaceutically active composition, whereby, for example, the component (b) can be distributed in the outer region (the portion that comes into contact with the release medium most quickly) of the matrix and the other component (a) is distributed inside the matrix. On contact with the release medium, the outer matrix layer initially (and rapidly) swells and firstly releases the NSAID component, followed by the significantly (more) retarded release of component (a). Examples of a suitable matrix include matrices with 1 to 80 % by weight of one or more hydrophilic or hydrophobic polymers as pharmaceutically acceptable matrix formers. A further example of a suitable matrix may be inferred from US 4,389,393 the respective contents of which hereby being incorporated by reference and forming part of the disclosure of the present invention.

The amount of the inventive pharmaceutically active combination, salt, or compound to be administered to the patient may vary depending on different factors well known to those skilled in the art, for example, the weight of the patient, the route of administration, or the severity of the illness.

In a further aspect the present invention relates to the use of an inventive combination as described herein and/or a pharmaceutical salt as described herein and/or a compound of formula (I") as described herein for the preparation of a medicament for the treatment of pain.

In another embodiment the present invention relates to the use of an inventive combination as described herein and/or a pharmaceutical salt as described herein and/or a compound of formula (I") as described herein for the preparation of a medicament for the treatment of pain, wherein the pain is selected from inflammatory pain, neuropathic pain, acute pain, chronic pain, visceral pain, migraine pain and cancer pain.

In yet another aspect the present invention relates to a method of treating pain in a mammal, preferably a human, which comprises administering an effective amount of an inventive combination as described herein and/or a pharmaceutical salt as described herein and/or a compound of formula (I") as described herein to the mammal.

In a further aspect of the present invention it relates to a method of treating pain in a mammal, preferably a human, which comprises administering an effective amount of an inventive combination as described herein and/or a pharmaceutical salt as described herein and/or a compound of formula (I") as described herein to the mammal, wherein the pain is selected from inflammatory pain, neuropathic pain, acute pain, chronic pain, visceral pain, migraine pain and cancer pain.

### Pharmacological methods:

### A. Randall-Selitto test in rats

The weight ratios of the components (a) and (b) that will lead to a supra-additive effect (synergistic effect) of the inventive pharmaceutical composition may be determined via the test of Randall and Selitto as described in Arch. lnt. Pharmacodyn., 1957, 111: 409 to 419, which is a model for inflammatory pain. The respective part of the literature is hereby incorporated by reference and forms part of the present disclosure.

By means of injection of 0.1 ml of Carrageenin-suspension ventrally into a hind paw of a rat an oedema is induced, on which pain is generated 4 hours later by continuously increasing pressure with a stamp (2 mm tip diameter). The antinociceptive and antihyperalgesic activity of the tested substance is determined at different points in time after administration of the substance. The measured value to be determined and at the same time also the end point of the pain test is the pressure at which the vocalisation reaction of the rat occurs. The percentage maximum possible effect (%MPE) is calculated. The maximum pressure of the stamp is 250 g. The group size is n = 10.

The analysis of the results with respect to a supra-additive effect of the inventive pharmaceutical composition comprising the components (a) and (b) is carried out via statistical comparison of the theoretical additive ED₅₀-value with the experimentally determined ED₅₀-value of a so-called fixed ratio combination (isobolographic analysis according to Tallarida JT, Porreca F, and Cowan A. Statistical analysis of drug-drug and site-site interactions with isobolograms. Life Sci 1989; 45: 947 - 961).
The interactions studies presented herein were performed using equieffective doses of the two components, calculated from the ratio of the respective ED₅₀ values of the components if administered alone.

### A.

The application route was intravenous (i.v.) for (1R,2R)-3-(3-Dimethylamino-1-ethyl-2-methyl-propyl)-phenol (A) and intraperitoneal (i.p.) for the NSAIDs Diclofenac-sodium and Ibuprofen. When A was applied alone, the peak effect was reached 15 min p. appl. (timepoint of first measurement) and an ED₅₀-value of 1.878 (1.694-2.065) mg/kg i.v. was calculated. Diclofenac-Sodium and Ibuprofen induced dose-dependent analgesic effects with an ED₅₀-value of 145.4 (134.4-154.6) or 139.1 (128.3 - 148.9) mg/kg i. p. respectively, reaching the peak effect 30 min p. appl. According to their respective timepoint of peak effect, (1 R,2R)-3-(3-Dimethylamino-1-ethyl-2-methyl-propyl)-phenol was applied 15 min and Diclofenac-Sodium and Ibuprofen 30 min before timepoint of measurement of the interaction-experiments (i. e. Diclofenac-Sodium or Ibuprofen were applied 15 min before (1 R,2R)-3-(3-Dimethylamino-1-ethyl-2-methyl-propyl)-phenol, respectively). Thus, the time point of ED₅₀ calculation of the combination corresponds to the timepoint of the peak effect of the respective compound. The isobolographic analysis revealed that the experimental ED₅₀-values of the combinations were significantly lower than the respective theoretical ED₅₀-values. Thus, the combination studies demonstrate significant synergistic interaction of (1 R,2R)-3-(3-Dimethylamino-1-ethyl-2-methylpropyl)-phenol with both NSAIDs, Diclofenac-Sodium and Ibuprofen.

The results of the isobolographic analysis are summarized in the following table. Experimental ED₅₀ values of Diclofenac-Sodium, A and Ibuprofen and isobolographic analysis of the interaction between A with Diclofenac-Sodium or Ibuprofen, respectively.

**Table 1:**

| Substance / ED₅₀ [mg/kg] (confidence interval) | A | Ibuprofen | Diclofenac -Sodium | Theoretical ED₅₀ of the combination | Experimental ED₅₀ of the combination | interaction |
|---|---|---|---|---|---|---|
| A + Ibuprofen | 1,878 (1,694 - 2,065)* | 139,1 (128,3 - 148,9) | - | -74,74) | 70, 45 (66, 16 35, 51 (31, 46 -39,54) | supraadditive (p < 0.001) |
| A + Diclofenac- Sodium | 1,878 (1,694 - 2,065)* | - | 145,4 (134,4 - 154,6) | 73,64 (69,24 - 78,04) | 36,00 (30,58 - 40,63) | supraadditive (p < 0.001) |

| | | | | | | |
|---|---|---|---|---|---|---|
| *: identical single-substance groups with A for both combinations p: level of statistical significance. | | | | | | |

From the table 1 given above, the ratio of A and Diclofenac-Sodium can be calculated to be 1:77.5, the ratio of A to Ibuprofen to be 1:73.8.

### B.

The application route was intravenous (i.v.) for (1R,2R)-3-(3-Dimethylamino-1-ethyl-2-methyl-propyl)-phenol (A) and intraperitoneal (i.p.) for the NSAIDs (+)-Naproxen, Ketoprofen and Metamizol-Sodium. When A was applied alone, the peak effect was reached 15 min p. appl. (timepoint of first measurement) and an ED₅₀-value of 1.88 (1.70-2.07) mg/kg i.v. was calculated. (+)-Naproxen, Ketoprofen and Metamizol sodium induced dose-dependent analgesic effects with an ED₅₀-value of 164 (158-169), 224 (210 - 237) and 88.1 (77.5 - 98.3) mg/kg i. p. respectively, reaching the peak effect 45 min p. appl. According to their respective timepoint of peak effect, (1 R,2R)-3-(3-Dimethylamino-1-ethyl-2-methyl-propyl)-phenol was applied 15 min and (+)-Naproxen, Ketoprofen and Metamizol-Sodium 45 min before timepoint of measurement of the interaction-experiments (i. e. (+)-Naproxen, Ketoprofen and Metamizol-Sodium were applied 30 min before (1R,2R)-3-(3-Dimethylamino-1-ethyl-2-methyl-propyl)-phenol, respectively). Thus, the time point of ED₅₀ calculation of the combination corresponds to the timepoint of the peak effect of the respective compound. The combination studies demonstrate significant synergistic interaction of (1R,2R)-3-(3-Dimethylamino-1-ethyl-2-methyl-propyl)-phenol with both NSAIDs Ketoprofen and Metamizol sodium and additive interaction of (1 R,2R)-3-(3-Dimethylamino-1-ethyl-2-methyl-propyl)-phenol and Naproxen.

The results of the isobolographic analysis are summarized in the following table 2.

Table 2: Experimental ED₅₀ values of A, (+)-Naproxen, Ketoprofen and Metamizol-Sodium and isobolographic analysis of the interaction between A with (+)-Naproxen, Ketoprofen and Metamizol-Sodium, respectively.

| Substance /ED₅₀ [mg/kg] (confidence interval) | A | (+)-Naproxen | Ketoprofen | Metamizol- Sodium | Theoretical ED₅₀ of the combination | Experimental ED₅₀ of the combination |
|---|---|---|---|---|---|---|
| A + (+)-Naproxen (REFERENZ) | 1.88 (1.70-2.07)* | 164 (158-169) | - | - | 82.6(78.3-86.9) | 79,3 (72.4-87.0)¹⁾ |
| A+ Ketoprofen | 1,88 (1.70-2.07)* | - | 224(210-237) | - | 113(106-119) | 91.4(82.9-98.9)²⁾ |
| A + Metamizol-Sodium | 1.88 (1.70-2.07)* | - | - | 88.1 (77.5 - 98.3) | 45.0 (41.6 -48.4) | 38.8 (35.3-41.9)²⁾ |

| | | | | | | |
|---|---|---|---|---|---|---|
| 1) additive interaction (p < 0.001) 2) supraadditive interaction (p < 0.001) *: identical single-substance groups with A for all three combinations p: level of statistical significance | | | | | | |

The ratio of A and (+)-Naproxen used in the experiments was 1:87.3, the ratio of A and Ketoprofen 1:119 and the ratio of A and Metamizol-Sodium was 1 :46.9.

The following examples are intended to clarify the invention

### Examples:

### REFERENCE EXAMPLE 1. Preparation of 4-butyl-1,2-diphenylpyrazolidine-3,5-dione with 3-((2R,3R)-1-(dimethylamino)-2-methylpentan-3-yl)phenol(1:1)

3-((2R,3R)-1-(Dimethylamino)-2-methylpentan-3-yl)phenol (250 mg) was dissolved white heating in as little ethanol as possible. 4-Butyl-1,2-diphenylpyrazolidine-3,5-dione (Phenylbutazone, 339 mg) was dissolved in H₂O/ethanol under heating. The solution were mixed, heated to reflux for 12 hours and allowed to cool to room temperature overnight. The solvent was removed in vacuo and the residue was dried by freeze drying to obtain a white solid (589 mg).

### 2. Preparation of S-((2R,3R)-1-(dimethylamlno)-2-methylpentan-3-yl)phenyl 2-(3-benzoylphenyl)propanoate

2-(3-Benzoylphenyl)propanoic acid (Ketoprofen, 1.04 g, 4.275 mmol) was dissolved in dichloromethane (15 mL). 4,4-Dimethylaminopyridine (47.3 mg, 0.387 mmol) and 3-((2R,3R)-1-(dimethylamino)-2-methylpentan-3-yl)phenol(1.0 g, 4.5 mmol) were added. The solution was cooled to 0°C and dicyclohexylcarbodilmide (1.3 g, 6.3 mmol) in dichloromethane (5 mL) was added. The solution was stirred for 15 min at 0 °C followed by stirring for 48 hours at ambient temperature. The reaction mixture was filtered, the filtrate was washed with 0.5 M aqueous hydrochloric acid and aqueous NaHCO₃ (10 %), dried over sodium sulfate and the solvent was removed in vacuo to obtain a white solid (2.16g) which was further purified by conventional chromatographic methods.

### REFENCE EXAMPLE 3. Preparation of 3-((2R,3R)-1-(dimethylamino)-2-methylpentan-3-yl)phenyl2-(2-fluorobiphenyl-4-yl)propanoate

2-(2-Fluorobiphenyl-4-yl)propanoic add (Flurbiprofen,1.04 g, 4.275 mmol) was dissolved in dichloromethane (15 mL). 4,4-Dimethylaminopyridine (47.3 mg, 0.387 mmol) and 3-((2R,3R)-1-(dimethylamino)-2-methylpentan-3-yl)phenol (1.0 g, 4.5 mmol) were added. The solution was cooled to 0 °C and dicyclohexylcarbodiimide (1.3 g, 6.3 mmol) in dichloromethane (5 mL) was added. The solution was stirred for 15 min at 0 °C followed by stirring for 48 hours at ambient temperature. The reaction mixture was filtered, the filtrate was washed with 0.5 M aqueous hydrochloric acid and aqueous NaHCO₃ (10 %), dried over sodium sulfate and the solvent was removed in vacuo to obtain a white solid (2.13 g) which was further purified by conventional chromatographic methods.

### REFENCE EXAMPLE 4. Preparation of 4-hydroxy-2-methyl-N-2-pyridinyl-2H-1,2-benzothiazine-3-carboxamide-1,1-dioxide with 3-((2R,3R)-1-(dimethylamino)-2-methylpentan-3-yl)phenol (1:1)

4-Hydroxy-2-methyl-N-2-pyridinyl-2H-1,2-benzothiazine-3-carboxamide-1,1-dioxide (Piroxicam, 302 mg) and 3-((2R,3R)-1-(dimethylamino)-2-methylpentan-3-yl)phenol (200 mg) were dissolved in a little amount of acetone. The resulting mixture was heated at 40°C overnight and stirred 24 hours at ambient temperature. The solvent was removed in vacuo and the residue was dried by freeze drying to obtain a colorless oil (517 mg).

### REFERENCE EXAMPLE 5. Preparation of (2R,3R)-3-(3-hydroxyphenyl)-N,N,2-trimethylpentan-1-aminium 2-(2-fluorobiphenyl-4-yl)propanoate (1:1)

2-(2-Fluorobiphenyl-4-yl)propanoic acid (Flurbiprofen, 220 mg, 0.903 mmol) and 3-((2R,3R)-1-(dimethylamino)-2-methylpentan-3-yl)phenol (200 mg, 0.903 mmol) were dissolved in a little amount of acetone. The resulting mixture was heated at 40 °C for 13 hours and stirred at ambient temperature overnight. The solvent was removed in vacuo and the residue was dried to obtain a foam (450 mg).

### REFENCE EXAMPLE 6. Preparation of 4-hydroxy-2-methyl-N-(5-methyl-3-isoxazolyl)-2H-1,2-benzothiazine-3-carboxamide-1,1dioxide with 3-((2R,3R)-1-(dimethylamino)-2-methylpentan-3-yl)phenol (1:1)

4-Hydroxy-2-methyl-N-(5-methyl-3-isoxazolyl)-2H-1,2-benzothiazine-3-carboxamide-1,1-dioxide (Isoxicam, 687 mg) and 3-((2R,3R)-1-(dimethylamino)-2-methylpentan-3-yl)phenol (600 mg) were dissolved in a little amount of acetone. The resulting mixture was heated at 40 °C overnight and stirred 24 hours at ambient temperature. The solvent was removed in vacuo and the residue was dried by freeze drying to obtain a solid (350 mg).

### 7. Preparation of (2R,3R)-3-(3-hydroxyphenyl)-N,N,2-trimethylpentan-1-aminium 2-(3-benzoylphenyl)propanoate (1;1)

2-(3-Benzoylphenylpropanoic acid (Ketoprofen, 675 mg) and 3-((2R,3R)-1-(dimethylamino)-2-methylpentan-3-yl)phenol (500 mg) were dissolved in a little amount of acetone. The resulting mixture was stirred at rt for one hour, heated to 40 °C for 4 hours and allowed to cool to ambient temperature overnight. The solvent was removed in vacuo and the residue was dried by freeze drying to obtain a solid (740 mg).

### 8. Preparation of 3-((2R,3R)-1-(dimethylamino)-2-methylpentan-3-yl)phenyl2-(3-(2,6-dichlorophonylamino)phenyl)acetate

2-(3-(2,6-Dichlorophenylamino)phenyl)acetic acid (Diclofenac, 761 mg, 2.57 mmol) was dissolved in dichloromethane (15 mL). 4,4-Dimethylaminopyridine (27.6 mg, 0.23 mmol) and 3-((2R,3R)-1-(dimethylamino)-2-methylpentan-3-yl)phenol(600 mg, 2.71 mmol) were added. The solution was cooled to 0 °C and dicyclohexylcarbodiimide (759 mg, 3.79 mmol) in dichloromethane (5 mL) was added. The solution was stirred for 15 min at 0 °C followed by stirring for 48 hours at ambient temperature. The reaction mixture was filtered, the filtrate was washed with 0.5 M aqueous hydrochloric acid and aqueous NaHCO₃ (10 %), dried over sodium sulfate and the solvent was removed in vacuo to obtain a white solid (1.40 g) which was further purified by conventional chromatographic methods.

### REFERECE EXAMPLE 9. Preparation of 3-((2R,3R)-1-(dimethylamino)-2-methylpentan-3yl)phenyl 2-(1-(4-chlorobenzoyl)-5-methoxy-2-methyl-1H-indol-3-yl)acetate

2-(1-(4-Chlorobenzoyl)-5-methoxy-2-methyl-1H-indol-3-yl)acetic acid (indometazin, 1.53 g, 4.275 mmol) was dissolved in dichloromethane (15 mL). 4,4-Dimethylaminopyridine (47.3 mg, 0.387 mmol) and 3-((2R,3R)-1(dimethylamino)-2-methylpentan-3-yl)phenol (1.0 g, 4.5 mmol) were added. The solution was cooled to 0°C and dicyclohexylcarbodiimide (1.3 g, 6.3 mmol) in dichloromethane (5 mL) was added. The solution was stirred for 15 min at 0°C followed by stirring for 48 hours at ambient temperature. The reaction mixture was filtered, the filtrate was washed with 0.5 M aqueous hydrochloric acid and aqueous NaHCO₃ (10 %), dried over sodium sulfate and the solvent was removed in vacuo to obtain a white solid (1.84 g) which was further purified by conventional chromatographic methods.

### REFERENCE EXAMPLE 10. Preparation of (S)-3-((2R,3R)-1-(dimethylamino)-2-methylpentan-3-yl)phenyl 2-(6-methoxynaphthalen-2-yl)propanoate

(S)-2-(6-Methoxynaphthaten-2-yl)propanoic acid ((S)-(+)-Naproxen, 0.98 g, 4.275 mmol) was dissolved in dichloromethane (15 mL). 4,4-Dimethylaminopyridine (47,3 mg, 0.387 mmol) and 3-((2R,3R)-1-(dimethylamino)-2-methylpentan-3-yl)phenol (1.0 g, 4.5 mmol) were added. The solution was cooled to 0 °C and dicyclohexylcarbodiimide (1.3 g, 6.3 mmol) In dichloromethane (5 mL) was added. The solution was stirred for 15 min at 0°C followed by stirring for 48 hours at ambient temperature. The reaction mixture was filtered, the filtrate was washed with 0.5 M aqueous hydrochloric acid and aqueous NaHCO₃ (10%), dried over sodium sulfate and the solvent was removed in vacuo to obtain a white solid (1.54 g) which was further purified by conventional chromatographic methods.

### 11. Preparation of (2S)-3-(1-(dimethylamino)-2-methylpentan-3-yl)phenyl 2-(4-isobutylphenyl)propanoate

(R)-2-(4-Isobutylphenyl)propanoic acid (Ibuprofen, 881 mg, 4.275 mmol) was dissolved in dichloromethane (15 mL). 4,4-Dimethylaminopyridine (47.3 mg, 0.387 mmol) and 3-((2R,3R)-1-(dimethylamino)-2-methylpentan-3-yl)phenol (1.0 g, 4.5 mmol) were added. The solution was cooled to 0°C and dicyclohexylcarbodiimide (1.3 g, 6.3 mmol) in dichloromethane (5 mL) was added. The solution was stirred for 15 min at 0°C followed by stirring for 48 hours at ambient temperature. The reaction mixture was filtered, the filtrate was washed with 0.5 M aqueous hydrochloric acid and aqueous NaHCO₃ (10 %), dried over sodium sulfate and the solvent was removed in vacuo to obtain a white solid (2.0 g) which was further purified by conventional chromatographic methods.

### 12. Preparation of 3-(1-(dimethylamino)-2-methylpentan-3-yl)phenyl 2-(4-isobutylphenyl)propanoate

2-(4-Isobutylphenyl)propanoic acid (Ibuprofen, 881 mg, 4.275 mmol) was dissolved in dichloromethane (15 mL). 4,4-Dimethylaminopyridine (47.3 mg, 0.387 mmol) and 3-(1-(dimethylamino)-2-methylpentan-3-yl)phenol (1.0 g, 4.5 mmol) were added. The solution was cooled to 0 °C and dicyclohexylcarbodiimide (1.4 g, 6.8 mmol) in dichloromethane (5 mL) was added. The solution was stirred for 15 min at 0 °C followed by stirring for 48 hours at ambient temperature. The reaction mixture was filtered, the filtrate was washed with 0.5 M aqueous hydrochloric acid and aqueous NaHCO₃ (10 %), dried over sodium sulfate and the solvent was removed in vacuo to obtain a white solid (1.54 g) which was further purified by conventional chromatographic methods.

### REFERENCE EXAMPLE 13. Preparation of 3-(1-(dimethylamino)-2-methylpentan-3-yl)phenyl2-acetoxybenzoate

2-(Chlorocarbonyl)phenyl acetate (10 g, 50 mmol) and 3-(1-(dimethylamino)-2-methylpentan-3-yl)phenol (4.4 g, 19.9 mmol) in dichloromethane (40 mL) were stirred overnight at ambient temperature. The solvent was removed in vacuo and methylethylketone (40 mL), H2O (0.4 mL), trimethylchlorosilane (2.8 mL) and tert-butylmethylether were added. The solvent is removed in vacuo and a precipitate formed which was filtered off. The filtrate was further reduced in vacuo to obtain a white solid (4.0 g).

### 14. Preparation of 3-(1-(dimethylamino)-2-methylpentan-3-yl)phenol((1,5-dimethyl-3-oxo-2-phenylpyrazolidin-4-yl)(methyl)amino)methanesulfonate

Sodium ((1,5-dimethyl-3-oxo-2-phenylpyrazolidin-4-yl)(methyl)amino)methanesulfonate (Dipyrone (Metamizol), 260.6 mg, 0.77 mmol) and 3-(1-(dimethylamino)-2-methylpentan-3yl)phenol (200 mg, 0.77 mmol) were dissolved in a little amount of acetone. The resulting mixture was heated at 40 °C overnight and allowed to cool to room temperature. The solvent was removed in vacuo, acetone was added, and the resulting precipitate was filtered off to obtain a white solid (0.3 g).

### 15. Preparation of 3-(3-hydroxyphenyl)-N,N,2-trimethylpentan-1-aminium 2-acetoxybenzoate

2-Acetoxybenzoic acid (405 mg, 2.5 mmol) was dissolved in water. 3-(1-(Dimethylamino)-2-methylpentan-3-yl)phenol (500 mg, 2.5 mmol) was dissolved in ethanol while heating. Both solutions were mixed together and heated to reflux for 4 hours. The solvent was removed in vacuo to obtain a white solid (0.85 g).

### REFENCE EXAMPLE 16. Preparation of 3-(3-hydroxyphenyl)-N,N,2-trimethylpentan-1-aminium 2-(6-methoxynaphthalen-2-yl)propanoate

2-(6-Methoxynaphthalen-2-yl)propanoic acid (Naproxen, 518.1 mg, 2.25 mmol) was dissolved in water. 3-(1-(Dimethylamino)-2-methytpentan-3-yl)phenol (500 mg, 2.25 mmol) was dissolved in ethanol while heating. Both solutions were mixed together and heated to reflux for 7 hours. The solvent was removed in vacuo to obtain a colorless oil which was dissolved in a little amount of acetone and hexanes. After cooling to 4 °C a precipitate formed which was filtered off.

### 17. Preparation of (2R)-3-(3-hydroxyphenyl)-N,N,2-trimethylpentan-1-aminium (R)-2-(4-isobutylphenyl)propanoate

(R)-2-(4-Isobutylphenyl)propanoic acid (464 mg, 2.25 mmol) was dissolved in acetone (1.7 mL) and heated to 40°C for 10 minutes. 3-(1-(Dimethylamino)-2-methylpentan-3-yl)phenol (500 mg, 2.25 mmol) was added, the reaction mixture was heated to 40°C for 6 hours and allowed to cool to room temperature overnight. The solvent was reduced in vacuo and cooled to 4 °C. A precipitate formed which was filtered off to obtain the desired product (350 mg).

### 18. Preparation of 3-(3-hydroxyphenyl)-N,N,2-trimethylpentan-1-aminium 2-(2-(2,6-dichlorophenylamino)phenyl)acetate

Diclofenac (267.6 mg, 0.9 mmol) and 3-(1-(Dimethylamino)-2-methylpentan-3-yl)phenol (200 mg, 0.9 mmol) were dissolved in acetone (0.7 mL) while heating to 40 °C overnight. The solvent was removed in vacuo, acetone was added again and the reaction mixture was allowed to crystallise overnight at 4°C. A precipitate formed which was filtered off and dried to obtain the desired product (125 mg).

### Examples of pharmaceutical dosage forms:

### 19. Tablet comprising Diclofenac-Sodium and (1R,2R)-3-(3-Dimethylamino-1-ethyl-2-methyl-propyl)-phenol

Tablets comprising (1R,2R)-3-(3-Dimethylamino-1-ethyl-2-methyl-propyl)-phenol and Diclofenac-Sodium are manufactured in form of 3-layer tablets in order to prevent the formation of a salt formed from said components.

The layers of the multilayer tablet according to the invention are first produced individually. For this purpose the layer comprising (1R,2R)-3-(3-Dimethylamino-1-ethyl-2-methyl-propyl)-phenol hydrochloride was prepared by mixing the compound together with microcrystalline cellulose, highly dispersed silicon dioxide and magnesium stearate in a cube mixer. The separating layer was prepared by mixing microcrystalline cellulose, highly dispersed silicon dioxide and magnesium stearate in a cube mixer. The layer containing Diclofenac-Sodium was prepared by mixing micronised Diclofenac-Sodium, microcrystalline cellulose, highly dispersed silicon dioxide and magnesium stearate in a cube mixer. The two layers containing the active substances together with the interposed separating layer were then compressed in one step to form a three-layer tablet having a diameter of 12 mm. For this purpose the successive layer amounts were respectively slightly compressed in an eccentric tableting machine, following which the whole layer sequence was compressed.

### Composition of a 3-layer tablet

| | |
|---|---|
| 1st layer: 250 mg | |
| (1R,2R)-3-(3-Dimethylamino-1-ethyl-2-methyl-propyl)-phenol-HCl | 100.00 mg |
| Microcrystalline cellulose | 145.00 mg |
| (Avicel PH 101, FMC) | |
| Highly dispersed silicon dioxide | 2.50 mg |
| (Aerosil, Degussa) | |
| Magnesium stearate | 2.50 mg |
| | |
| Separating layer: 100 mg | |
| Microcrystalline cellulose | 98.00 mg |
| (Avicel PH 101, FMC) | |
| Highly dispersed silicon dioxide | 1.00 mg |
| (Aerosil, Degussa) | |
| Magnesium stearate | 1.00 mg |
| | |
| 3rd layer: 250 mg | |
| Diclofenac-Sodium, micronised | 50.00 mg |
| Microcrystalline cellulose | 195.00 mg |
| (Avicel PH 101, FMC) | |
| Highly dispersed silicon dioxide | 2.50 mg |
| (Aerosil, Degussa) | |
| Magnesium stearate | 2.50 mg |
| | |
| Total | 600.00 mg |

### 20. Preparation of a capsule comprising (1R,2R)-3-(3-Dimethylamino-1-ethyl-2-methyl-propyl)-phenol and Ketoprofen

| Composition | |
|---|---|
| Ketoprofen | 1000 g |
| (1R,2R)-3-(3-Dimethylamino-1-ethyl-2-methyl-propyl)-phenol | 500 g |
| Microcrystalline Cellulose | 500 g |
| Lactose monohydrate | 475 g |
| Magnesium stearate | 25 g |

All compounds are sieved through a 1 mm sieve, blended in a tumble mixer and filled into size 1 hard gelatin capsules with a filling weight of 250 mg.

### 21. Preparation of a tablet comprising (1R,2R)-3-(3-Dimethylamino-1-ethyl-2-methyl-propyl)-phenol and Ibuprofen

| Composition | |
|---|---|
| Ibuprofen | 2000 g |
| (1R,2R)-3-(3-Dimethylamino-1-ethyl-2-methyl-propyl)-phenol | 500 g |
| Lactose monohydrate | 450 g |
| Microcrystalline Cellulose | 1500 g |
| Sodium starch glycolate | 500 g |
| Magnesium stearate | 50 g |

All compounds are sieved through a 1 mm sieve, blended in a tumble mixer and compressed on an Korsch EKO tablet press into tablets of 12 mm diameter and a weight of 500 mg.

## Claims

1. A combination comprising as component(s):
(a) at least one 3-(3-Dimethylamino-1-ethyl-2-methyl-propyl)-phenol of formula (I), 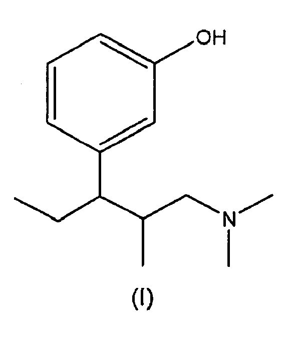 optionally in form of one of its pure stereoisomers, in particular an enantiomer or a diastereomer, a racemate or in form of a mixture of its stereoisomers, in particular enantiomers and/or diastereomers in any mixing ratio, or any corresponding acid addition salt thereof, or any solvate thereof, and
(b) one or more non-steroidal anti-inflammatory drugs (NSAIDs) selected from the group consisting of Diclofenac, Diclofenac-Sodium, Dipyrone (Metamizol), Metamizol-Sodium, Ibuprofen, Ketoprofen, (+)-Ibuprofen and (-)-Ibuprofen.

2. Combination according to claim 1, **characterized in that** component (a) is selected from
(1R,2R)-3-(3-Dimethylamino-1-ethyl-2-methyl-propyl)-phenol,
(1S,2S)-3-(3-Dimethylamino-1-ethyl-2-methyl-propyl)-phenol,
(1R,2S)-3-(3-Dimethylamino-1-ethyl-2-methyl-propyl)-phenol,
(1S,2R)-3-(3-Dimethylamino-1-ethyl-2-methyl-propyl)-phenol, and any mixture thereof.

3. Combination according to claim 2, **characterized in that** component (a) is selected from
(1R,2R)-3-(3-Dimethylamino-1-ethyl-2-methyl-propyl)-phenol,
(1S,2S)-3-(3-Dimethylamino-1-ethyl-2-methyl-propyl)-phenol, and any mixture thereof.

4. Combination according to claim 2 or 3, **characterized in that** component (a) is the compound (1R,2R)-3-(3-Dimethylamino-1-ethyl-2-methyl-propyl)-phenol of formula (I'), 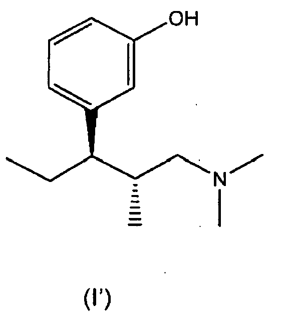 or an acid addition salt thereof, whereby the acid addition salt of hydrochloride is preferred.

5. Combination according to any of claims 1-4, **characterized in that** component (b) is selected from the group consisting of Diclofenac, Diclofenac-Sodium, Ibuprofen, Metamizol, Metamizol-Sodium and Ketoprofen.

6. Combination according to any of claims 1-5, **characterized in that** component (b) is selected from the group consisting of Diclofenac, Diclofenac-Sodium and Ibuprofen.

7. Combination according to any of claims 1-6, **characterized in that** components (a) and (b) are present as a salt formed from these two components.

8. Combination according to any of claims 1-7, **characterized in that** components (a) and (b) are present in such a weight ratio that the composition will exert a synergistic effect upon administration to a patient.

9. A pharmaceutical salt, **characterized in that** the cationic salt component is formed from
(a) at least one compound 3-(3-Dimethyfamino-1-ethyl-2-methyl-propyl)-phenol of formula (I), 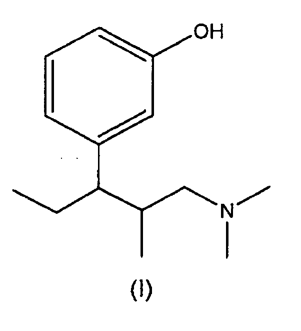 optionally in form of one of its pure stereoisomers, in particular an enantiomer or a diastereomer, a racemate or in form of a mixture of its stereoisomers, in particular enantiomers and/or diastereomers in any mixing ratio, or any solvate thereof,
and the anionic salt component is formed from
(b) one or more acidic non-steroidal anti-inflammatory drug selected from the group consisting of Diclofenac, Dipyrone (Metamizol), Ibuprofen, Ketoprofen, (+)-Ibuprofen and (-)-Ibuprofen.

10. A pharmaceutical salt according to claim 9, **characterized in that** component (a) is selected from
(1R,2R)-3-(3-Dimethylamino-1-ethyl-2-methyl-propyl)-phenol,
(1S,2S)-3-(3-Dimethylamino-1-ethyl-2-methyl-propyl)-phenol,
(1R,2S)-3-(3-Dimethylamino-1-ethyl-2-methyl-propyl)-phenol,
(1S,2R)-3-(3-Dimethylamino-1-ethyl-2-methyl-propyl)-phenol and any mixture thereof.

11. A pharmaceutical salt according to claim 10, **characterized in that** component (a) is selected from
(1 R,2R)-3-(3-Dimethylamino-1-ethyl-2-methyl-propyl)-phenol,
(1S,2S)-3-(3-Dimethylamino-1-ethyl-2-methyl-propyl)-phenol, and any mixture thereof.

12. A pharmaceutical salt according to claim 10 or 11, **characterized in that** component (a) is the compound (1 R,2R)-3-(3-Dimethylamino-1-ethyl-2-methylpropyl)-phenol of formula (I')

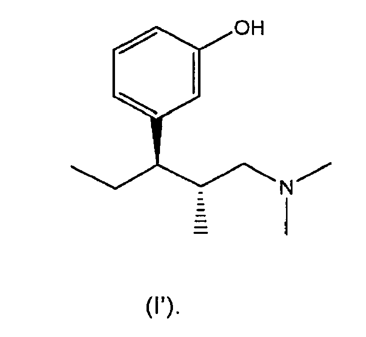

13. A pharmaceutically active salt according to any one of claims 9-12, **characterized in that** the acidic non-steroidal anti-inflammatory drug is selected from Diclofenac, Dipyrone (Metamizol), Ibuprofen and Ketoprofen.

14. A compound of general formula (I") 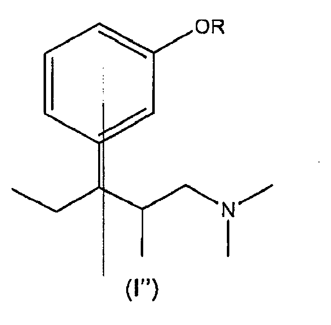 wherein R is a fragment of a non-steroidal anti-inflammatory drug (NSAID) that is attached to the oxygen atom via a covalent linkage,
optionally in form of one of its pure stereoisomers, in particular an enantiomer or a diastereomer, a racemate or in form of a mixture of its stereoisomers, in particular enantiomers and/or diastereomers in any mixing ratio, or any corresponding acid addition salt thereof, or any solvate thereof,
whereby said covalent linkage is obtained from the carboxy group of an NSAID selected from the group consisting of Diclofenac, Dipyrone (Metamizol), Ibuprofen, Ketoprofen, (+)-Ibuprofen and (-)-Ibuprofen.

15. A compound according to claim 14, **characterized in that** it is derived from
(1 R,2R)-3-(3-Dimethylamino-1-ethyl-2-methyl-propyl)-phenol,
(1S,2S)-3-(3-Dimethylamino-1-ethyl-2-methyl-propyl)-phenol,
(1 R,2S)-3-(3-Dimethylamino-1-ethyl-2-methyl-propyl)-phenol,
(1S,2R)-3-(3-Dimethylamino-1-ethyl-2-methyl-propyl)-phenol and any mixture thereof.

16. A compound according to claim 15, **characterized in that** it is derived from
(1 R,2R)-3-(3-Dimethylamino-1-ethyl-2-methyl-propyl)-phenol,
(1S,2S)-3-(3-Dimethylamino-1-ethyl-2-methyl-propyl)-phenol, and any mixture thereof.

17. A compound according to claim 15 or 16, **characterized in that** it is derived from the compound (1R,2R)-3-(3-Dimethylamino-1-ethyl-2-methyl-propyl)-phenol of formula (I')

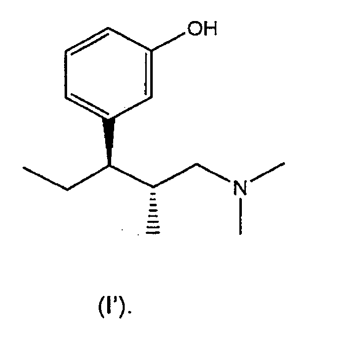

18. A compound according to any one of claims 14-17, **characterized in that** it is derived from an acidic non-steroidal anti-inflammatory drug selected from Diclofenac, Dipyrone (Metamizol), Ibuprofen and Ketoprofen.

19. A pharmaceutical composition comprising a combination according to any one of claims 1-8 and/or a pharmaceutical salt according to any one of claims 9-13 and/or a compound of formula (I") according to any one of claims 14-18 and optionally one or more auxiliary agents.

20. A dosage form comprising a combination according to any one of claims 1-8 and/or a pharmaceutical salt according to any one of claims 9-13 and/or a compound of formula (I") according to any one of claims 14-18 and optionally one or more auxiliary agents.

21. A dosage form according to claim 20, **characterized in that** it is suitable for oral, intravenous, intraperitoneal, intradermal, intrathekal, intramuscular, intranasal, transmucosal, subcutaneous, or rectal administration.

22. A dosage form according to claim 20 or 21, **characterized in that** one or both of the components (a) and (b) is/are present in controlled-release form and/or the pharmaceutical salt is present in controlled-release form and/or the compound of formula (I") is present in controlled-release form.

23. A dosage form according to any one of claims 20-22, **characterized in that** it comprises additionally caffeine.

24. Use of a combination according to any one of claims 1-8 and/or a pharmaceutical salt according to any one of claims 9-13 and/or a compound according to any one of claims 14-18 for the preparation of a medicament for the treatment of pain.

25. Use according to claim 24, **characterised in that** the pain is selected from inflammatory pain, neuropathic pain, acute pain, chronic pain, visceral pain, migraine pain and cancer pain.

26. Use according to claim 24 or 25, **characterised in that** the medicament is adapted for simultaneous or sequential administration, wherein compound (a) may be administered before or after compound (b) and wherein compounds (a) or (b) are administered either by the same or a different pathway of administration.

## Patentansprüche

1. Kombination, die als Komponente(n) Folgendes umfasst:
(a) wenigstens ein 3-(3-Dimethylamino-1-ethyl-2-methylpropyl)-phenol der Formel (I) 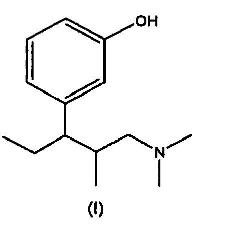 gegebenenfalls in Form von einem seiner reinen Stereoisomere, insbesondere einem Enantiomer oder einem Diastereomer, einem Racemat oder in Form eines Gemischs aus seinen Stereoisomeren, insbesondere Enantiomeren und/oder Diastereomeren in einem beliebigen Mischungsverhältnis, oder ein beliebiges entsprechendes Säureadditionsalz davon oder ein beliebiges Solvat davon, und
(b) ein oder mehrere nicht-steroidale entzündungshemmende Medikamente (NSAID), die ausgewählt sind aus der Gruppe bestehend aus Diclofenac, Diclofenac-Natrium, Dipyrone (Metamizol), Metamizol-Natrium, Ibuprofen, Ketoprofen, (+)-Ibuprofen und (-)-Ibuprofen.

2. Kombination nach Anspruch 1, **dadurch gekennzeichnet, dass** die Komponente (a) ausgewählt ist aus:
(1R,2R)-3-(3-Dimethylamino-1-ethyl-2-methylpropyl)-phenol,
(1S,2S)-3-(3-Dimethylamino-1-ethyl-2-methylpropyl)-phenol,
(1R,2S)-3-(3-Dimethylamino-1-ethyl-2-methylpropyl)-phenol,
(1S,2R)-3-(3-Dimethylamino-1-ethyl-2-methylpropyl)-phenol und einem beliebigen Gemisch davon.

3. Kombination nach Anspruch 2, **dadurch gekennzeichnet, dass** Komponente (a) ausgewählt ist aus:
(1R,2R)-3-(3-Dimethylamino-1-ethyl-2-methylpropyl)-phenol,
(1S,2S)-3-(3-Dimethylamino-1-ethyl-2-methylpropyl)-phenol und einem beliebigen Gemisch davon.

4. Kombination nach Anspruch 2 oder 3, **dadurch gekennzeichnet, dass** Komponente (a) die Verbindung (1R,2R)-3-(3-Dimethylamino-1-ethyl-2-methylpropyl)-phenol der Formel (I') 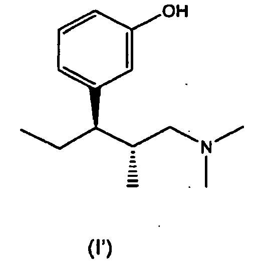 oder ein Säureadditionssalz davon ist, wobei das Säureadditionssalz von Hydrochlorid bevorzugt wird.

5. Kombination nach einem der Ansprüche 1-4, **dadurch gekennzeichnet, dass** Komponente (b) ausgewählt ist aus der Gruppe bestehend aus Diclofenac, Diclofenac-Natrium, Ibuprofen, Metamizol, Metamizol-Natrium und Ketoprofen.

6. Kombination nach einem der Ansprüche 1-5, **dadurch gekennzeichnet, dass** Komponente (b) ausgewählt ist aus der Gruppe bestehend aus Diclofenac, Diclofenac-Natrium und Ibuprofen.

7. Kombination nach einem der Ansprüche 1-6, **dadurch gekennzeichnet, dass** die Komponenten (a) und (b) als ein Salz vorliegen, das aus diesen beiden Komponenten gebildet ist.

8. Kombination nach einem der Ansprüche 1-7, **dadurch gekennzeichnet, dass** die Komponenten (a) und (b) in einem solchen Gewichtsverhältnis vorliegen, dass die Zusammensetzung einen synergistischen Effekt auf die Verabreichung an einen Patienten ergeben wird.

9. Pharmazeutisches Salz, **dadurch gekennzeichnet, dass** die kationische Salzkomponente gebildet ist aus
(a) wenigstens einer Verbindung 3-(3-Dimethylamino-1-ethyl-2-methylpropyl)-phenol der Formel (I) 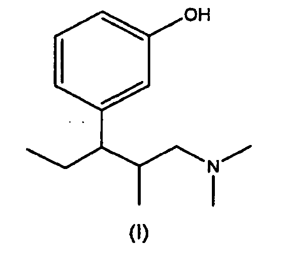 gegebenenfalls in Form von einem seiner reinen Stereoisomere, insbesondere einem Enantiomer oder einem Diastereomer, einem Racemat oder in Form eines Gemischs aus seinen Stereoisomeren, insbesondere Enantiomeren und/oder Diastereomeren in einem beliebigen Gemischverhältnis, oder einem beliebigen Solvat davon,
und die anionische Salzkomponente gebildet ist aus
(b) einem oder mehreren azidischen nicht-steroidalen entzündungshemmenden Medikamenten (NSAIDs), die ausgewählt sind aus der Gruppe bestehend aus Diclofenac, Dipyrone (Metamizol), Ibuprofen, Ketoprofen, (+)-Ibuprofen und (-)-Ibuprofen.

10. Pharmazeutisches Salz nach Anspruch 9, **dadurch gekennzeichnet, dass** Komponente (a) ausgewählt ist aus
(1R,2R)-3-(3-Dimethylamino-1-ethyl-2-methylpropyl)-phenol,
(1S,2S)-3-(3-Dimethylamino-1-ethyl-2-methylpropyl)-phenol,
(1R,2S)-3-(3-Dimethylamino-1-ethyl-2-methylpropyl)-phenol,
(1S,2R)-3-(3-Dimethylamino-1-ethyl-2-methylpropyl)-phenol und einem beliebigen Gemisch davon.

11. Pharmazeutisches Salz nach Anspruch 10, **dadurch gekennzeichnet, dass** Komponente (a) ausgewählt ist aus:
(1R,2R)-3-(3-Dimethylamino-1-ethyl-2-methylpropyl)-phenol,
(1S,2S)-3-(3-Dimethylamino-1-ethyl-2-methylpropyl)-phenol und einem beliebigen Gemisch davon.

12. Pharmazeutisches Salz nach Anspruch 10 oder 11, **dadurch gekennzeichnet, dass** Komponente (a) die Verbindung (1R,2R)-3-(3-Dimethylamino-1-ethyl-2-methylpropyl)-phenol der Formel (I') ist:

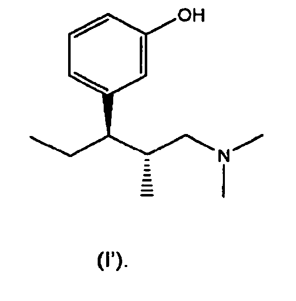

13. Pharmazeutisch aktives Salz nach einem der Ansprüche 9-12, **dadurch gekennzeichnet, dass** das azidische nicht-steroidale entzündungshemmende Medikament ausgewählt ist aus Diclofenac, Dipyrone (Metamizol), Ibuprofen und Ketoprofen.

14. Verbindung der allgemeinen Formel (I") 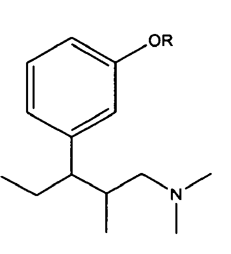 wobei R ein Fragment eines nicht-steroidalen entzündungshemmenden Medikaments (NSAID) ist, das über eine kovalente Bindung an das Sauerstoffatom angelagert ist,
gegebenenfalls in Form von einem seiner reinen Stereoisomere, insbesondere einem Enantiomer oder einem Diastereomers, einem Racemat oder in Form eines Gemischs aus seinen Stereoisomeren, insbesondere Enantiomeren und/oder Diastereomeren in einem beliebigen Mischungsverhältnis, oder ein beliebiges entsprechendes Säureadditionsalz davon oder ein beliebiges Solvat davon,
wobei die genannte kovalente Bindung von der Carboxygruppe eines NSAID erhalten wird, das ausgewählt ist aus der Gruppe bestehend aus Diclofenac, Dipyrone (Metamizol), Ibuprofen, Ketoprofen, (+)-Ibuprofen und (-)-Ibuprofen.

15. Verbindung nach Anspruch 14, **dadurch gekennzeichnet, dass** sie abgeleitet ist von:
(1R,2R)-3-(3-Dimethylamino-1-ethyl-2-methylpropyl)-phenol,
(1S,2S)-3-(3-Dimethylamino-1-ethyl-2-methylpropyl)-phenol,
(1R,2S)-3-(3-Dimethylamino-1-ethyl-2-methylpropyl)-phenol,
(1S,2R)-3-(3-Dimethylamino-1-ethyl-2-methylpropyl)-phenol und einem beliebigen Gemisch davon.

16. Verbindung nach Anspruch 15, **dadurch gekennzeichnet, dass** sie abgeleitet ist von:
(1R,2R)-3-(3-Dimethylamino-1-ethyl-2-methylpropyl)-phenol,
(1S,2S)-3-(3-Dimethylamino-1-ethyl-2-methylpropyl)-phenol und einem beliebigen Gemisch davon.

17. Verbindung nach Anspruch 15 oder 16, **dadurch gekennzeichnet, dass** sie abgeleitet ist von der Verbindung (1R,2R)-3-(3-Dimethylamino-1-ethyl-2-methylpropyl)-phenol der Formel (I')

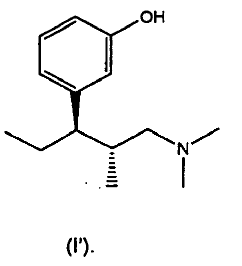

18. Verbindung nach einem der Ansprüche 14-17, **dadurch gekennzeichnet, dass** sie von einem azidischen nicht-steroidalen entzündungshemmenden Medikament abgeleitet ist, das ausgewählt wurde aus Diclofenac, Dipyrone (Metamizol), Ibuprofen und Ketoprofen.

19. Pharmazeutische Zusammensetzung, die eine Kombination nach einem der Ansprüche 1-8 und/oder ein pharmazeutisches Salz nach einem der Ansprüche 9-13 und/oder eine Verbindung der Formel (I") nach einem der Ansprüche 14-18 und gegebenenfalls ein oder mehrere Hilfsmittel umfasst.

20. Dosierungsform, die eine Kombination nach einem der Ansprüche 1-8 und/oder ein pharmazeutisches Salz nach einem der Ansprüche 9-13 und/oder eine Verbindung der Formel (I") nach einem der Ansprüche 14-18 und gegebenenfalls ein oder mehrere Zusatzmittel umfasst.

21. Dosierungsform nach Anspruch 20, **dadurch gekennzeichnet, dass** sie für eine orale, intravenöse, intraperitoneale, intradermale, intrathekale, intramuskuläre, intranasale, transmukosale, subkutane oder rektale Verabreichung geeignet ist.

22. Dosierungsform nach Anspruch 20 oder 21, **dadurch gekennzeichnet, dass** eine oder beide der Komponenten (a) und (b) in einer Form mit gesteuerter Freisetzung vorliegt/vorliegen und/oder das pharmazeutische Salz in einer Form mit gesteuerter Freisetzung vorliegt und/oder die Verbindung der Formel (I") in einer Form mit gesteuerter Freisetzung vorliegt.

23. Dosierungsform nach einem der Ansprüche 20-22, **dadurch gekennzeichnet, dass** sie zusätzlich Koffein umfasst.

24. Verwendung einer Kombination nach einem der Ansprüche 1-8 und/oder eines pharmazeutischen Salzes nach einem der Ansprüche 9-13 und/oder einer Verbindung nach einem der Ansprüche 14-18 für die Herstellung eines Medikaments zur Behandlung von Schmerzen.

25. Verwendung nach Anspruch 24, **dadurch gekennzeichnet, dass** der Schmerz ausgewählt ist aus Entzündungsschmerzen, neuropathischen Schmerzen, akuten Schmerzen, chronischen Schmerzen, viszeralen Schmerzen, Migräneschmerzen und Krebsschmerzen.

26. Verwendung nach Anspruch 24 oder 25, **dadurch gekennzeichnet, dass** das Medikament für eine simultane oder sequentielle Verabreichung adaptiert ist, wobei Verbindung (a) vor oder nach Verbindung (b) verabreicht werden kann und wobei die Verbindungen (a) und (b) entweder auf demselben oder über einen anderen Verabreichungsweg verabreicht werden können.

## Revendications

1. Combinaison comprenant au titre de composants :
(a) au moins un 3-(3-diméthylamino-1-éthyl-2-méthyl-propyl)-phénol de formule (I), 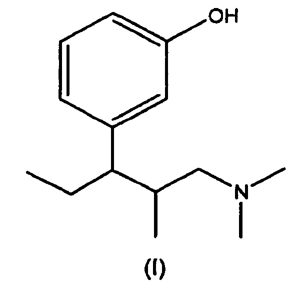 éventuellement sous forme de l'un de ses stéréoisomères purs, en particulier d'un énantiomère ou d'un diastéréoisomère, d'un racémate ou sous forme d'un mélange de ses stéréoisomères, en particulier de ses énantiomères et/ou de ses diastéréoisomères dans tout rapport de mélange, ou de l'un quelconque de ses sels d'addition d'acide, ou de l'un quelconque de ses solvates, et
(b) un ou plusieurs anti-inflammatoires non stéroïdiens (AINS) choisis dans le groupe constitué par les suivants : Diclofénac, Diclofénac-Sodium, Dipyrone (Métamizol), Métamizol-Sodium, Ibuprofène, Kétoprofène, (+)-Ibuprofène et (-)-Ibuprofène.

2. Combinaison selon la revendication 1, **caractérisée en ce que** le composant (a) est choisi parmi les suivants :
(1R,2R)-3-(3-diméthylamino-1-éthyl-2-méthyl-propyl)-phénol,
(1S,2S)-3-(3-diméthylamino-1-éthyl-2-méthyl-propyl)-phénol,
(1R,2S)-3-(3-diméthylamino-1-éthyl-2-méthyl-propyl)-phénol,
(1S,2R)-3-(3-diméthylamino-1-éthyl-2-méthyl-propyl)-phénol, et n'importe lequel de leurs mélanges.

3. Combinaison selon la revendication 2, **caractérisée en ce que** le composant (a) est choisi parmi les suivants :
(1R,2R)-3-(3-diméthylamino-1-éthyl-2-méthyl-propyl)-phénol,
(1S,2S)-3-(3-diméthylamino-1-éthyl-2-méthyl-propyl)-phénol, et n'importe lequel de leurs mélanges.

4. Combinaison selon la revendication 2 ou 3, **caractérisée en ce que** le composant (a) est le composé (1R,2R)-3-(3-diméthylamino-1-éthyl-2-méthyl-propyl)-phénol de formule (I'), 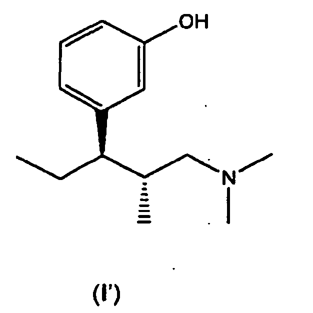 ou l'un de ses sels d'addition d'acide, le sel d'addition acide avec l'acide chlorhydrique étant préféré.

5. Combinaison selon l'une quelconque des revendications 1 à 4, **caractérisée en ce que** le composant (b) est choisi dans le groupe constitué par les suivants : Diclofénac, Diclofénac-Sodium, Ibuprofène, Métamizole, Métamizole-Sodium et Kétoprofène.

6. Combinaison selon l'une quelconque des revendications 1 à 5, **caractérisée en ce que** le composant (b) est choisi dans le groupe constitué par les suivants : Diclofénac, Diclofénac-Sodium et Ibuprofène.

7. Combinaison selon l'une quelconque des revendications 1 à 6, **caractérisée en ce que** les composants (a) et (b) sont présents sous forme d'un sel formé à partir de ces deux composants.

8. Combinaison selon l'une quelconque des revendications 1 à 7, **caractérisée en ce que** les composants (a) et (b) sont présents à un rapport massique tel que la composition exercera un effet synergique lors de l'administration à un patient.

9. Sel pharmaceutique, **caractérisé en ce que** le composant cationique du sel est formé à partir de :
(a) au moins un composé 3-(3-diméthylamino-1-éthyl-2-méthyl-propyl)-phénol de formule (I), 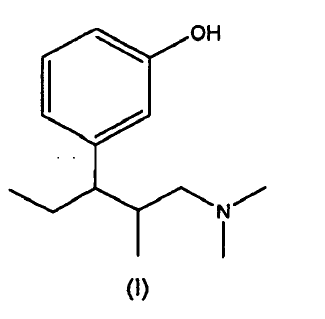 éventuellement sous forme de l'un de ses stéréoisomères purs, en particulier d'un énantiomère ou d'un diastéréoisomère, d'un racémate ou sous forme d'un mélange de ses stéréoisomères, en particulier de ses énantiomères et/ou de ses diastéréoisomères dans tout rapport de mélange, ou de l'un quelconque de ses solvates,
et **en ce que** le composant anionique du sel est formé à partir de :
(b) un ou plusieurs anti-inflammatoires non stéroïdiens acides choisis dans le groupe constitué par les suivants : Diclofénac, Dipyrone (Métamizol), Ibuprofène, Kétoprofène, (+)-Ibuprofène et (-)-Ibuprofène.

10. Sel pharmaceutique selon la revendication 9, **caractérisé en ce que** le composant (a) est choisi parmi les suivants :
(1R,2R)-3-(3-diméthylamino-1-éthyl-2-méthyl-propyl)-phénol,
(1S,2S)-3-(3-diméthylamino-1-éthyl-2-méthyl-propyl)-phénol,
(1R,2S)-3-(3-diméthylamino-1-éthyl-2-méthyl-propyl)-phénol,
(1S,2R)-3-(3-diméthylamino-1-éthyl-2-méthyl-propyl)-phénol, et n'importe lequel de leurs mélanges.

11. Sel pharmaceutique selon la revendication 10, **caractérisé en ce que** le composant (a) est choisi parmi les suivants :
(1R,2R)-3-(3-diméthylamino-1-éthyl-2-méthyl-propyl)-phénol,
(1S,2S)-3-(3-diméthylamino-1-éthyl-2-méthyl-propyl)-phénol, et n'importe lequel de leurs mélanges.

12. Sel pharmaceutique selon la revendication 10 ou 11, **caractérisé en ce que** le composant (a) est le composé (1R,2R)-3-(3-diméthylamino-1-éthyl-2-méthyl-propyl)-phénol de formule (I')

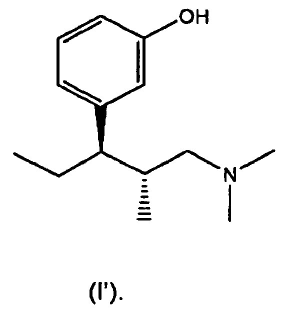

13. Sel pharmaceutiquement actif selon l'une quelconque des revendications 9 à 12, **caractérisé en ce que** l'anti-inflammatoire non stéroïdien acide est choisi parmi les suivants : Diclofénac, Dipyrone (Métamizol), Ibuprofène et Kétoprofène.

14. Composé de formule générale (I") 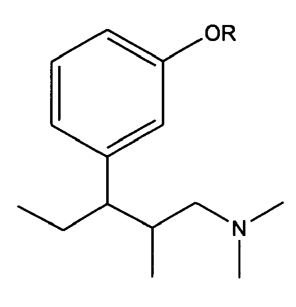 où R représente un fragment d'anti-inflammatoire non stéroïdien (AINS) lié à l'atome d'oxygène par une liaison covalente,
éventuellement sous forme de l'un de ses stéréoisomères purs, en particulier d'un énantiomère ou d'un diastéréoisomère, d'un racémate ou sous forme d'un mélange de ses stéréoisomères, en particulier de ses énantiomères et/ou de ses diastéréoisomères dans tout rapport de mélange, ou de l'un quelconque de ses sels d'addition d'acide, ou de l'un quelconque de ses solvates, ladite liaison covalente étant obtenue à partir du groupement carboxy d'un AINS choisi dans le groupe constitué par les suivants : Diclofénac, Dipyrone (Métamizol), Ibuprofène, Kétoprofène, (+)-Ibuprofène et (-)-Ibuprofène.

15. Composé selon la revendication 14, **caractérisé en ce qu'**il est dérivé des suivants :
(1R,2R)-3-(3-diméthylamino-1-éthyl-2-méthyl-propyl)-phénol,
(1S,2S)-3-(3-diméthylamino-1-éthyl-2-méthyl-propyl)-phénol,
(1R,2S)-3-(3-diméthylamino-1-éthyl-2-méthyl-propyl)-phénol,
(1S,2R)-3-(3-diméthylamino-1-éthyl-2-méthyl-propyl)-phénol, et n'importe lequel de leurs mélanges.

16. Composé selon la revendication 15, **caractérisé en ce qu'**il est dérivé des suivants :
(1R,2R)-3-(3-diméthylamino-1-éthyl-2-méthyl-propyl)-phénol,
(1S,2S)-3-(3-diméthylamino-1-éthyl-2-méthyl-propyl)-phénol, et n'importe lequel de leurs mélanges.

17. Composé selon la revendication 15 ou 16, **caractérisé en ce qu'**il est dérivé du composé (1R,2R)-3-(3-diméthylamino-1-éthyl-2-méthyl-propyl)-phénol de formule (I')

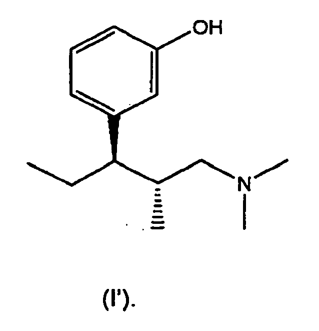

18. Composé selon l'une quelconque des revendications 14 à 17, **caractérisé en ce qu'**il est dérivé d'un anti-inflammatoire non stéroïdien acide choisi parmi les suivants : Diclofénac, Dipyrone (Métamizol), Ibuprofène et Kétoprofène.

19. Composition pharmaceutique comprenant une combinaison selon l'une quelconque des revendications 1 à 8 et/ou un sel pharmaceutique selon l'une quelconque des revendications 9 à 13 et/ou un composé de formule (I") selon l'une quelconque des revendications 14 à 18, et éventuellement un ou plusieurs agents auxiliaires.

20. Forme galénique comprenant une combinaison selon l'une quelconque des revendications 1 à 8 et/ou un sel pharmaceutique selon l'une quelconque des revendications 9 à 13 et/ou un composé de formule (I") selon l'une quelconque des revendications 14 à 18, et éventuellement un ou plusieurs agents auxiliaires.

21. Forme galénique selon la revendication 20, **caractérisée en ce qu'**elle est adaptée à une administration orale, intraveineuse, intrapéritonéale, intradermique, intrathécale, intramusculaire, intranasale, transmucosale, sous-cutanée ou rectale.

22. Forme galénique selon la revendication 20 ou 21, **caractérisée en ce que** l'un ou les deux composants (a) et (b) sont présents sous une forme à libération contrôlée et/ou **en ce que** le sel pharmaceutique est présent sous une forme à libération contrôlée et/ou **en ce que** le composé de formule (I") est présent sous une forme à libération contrôlée.

23. Forme galénique selon l'une quelconque des revendications 20 à 22, **caractérisée en ce qu'**elle comprend en outre de la caféine.

24. Emploi d'une combinaison selon l'une quelconque des revendications 1 à 8 et/ou d'un sel pharmaceutique selon l'une quelconque des revendications 9 à 13 et/ou d'un composé selon l'une quelconque des revendications 14 à 18 dans l'élaboration d'un médicament destiné au traitement de la douleur.

25. Emploi selon la revendication 24, **caractérisé en ce que** la douleur est choisie parmi la douleur inflammatoire, la douleur néuropathique, la douleur aiguë, la douleur chronique, la douleur viscérale, la
douleur liée à la migraine et la douleur liée au cancer.

26. Emploi selon la revendication 24 ou 25, **caractérisé en ce que** le médicament est adapté à une administration simultanée ou séquentielle, le composé (a) pouvant être administré avant ou après le composé (b), et les composés (a) ou (b) étant administrés par la même voie d'administration ou par une voie différente.
